(19) **Europäisches Patentamt**
**European Patent Office**
**Office européen des brevets**

(11) **EP 3 730 046 A1**

(12) # EUROPEAN PATENT APPLICATION
published in accordance with Art. 153(4) EPC

(43) Date of publication:
**28.10.2020 Bulletin 2020/44**

(51) Int Cl.:
***A61B 5/01*** (2006.01)

(21) Application number: **17935744.7**

(22) Date of filing: **28.12.2017**

(86) International application number:
**PCT/CN2017/119483**

(87) International publication number:
**WO 2019/119506 (27.06.2019 Gazette 2019/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **20.12.2017 CN 201711387804**

(71) Applicant: **Shenzhen Institutes of Advanced Technology**
**Shenzhen, Guangdong 518055 (CN)**

(72) Inventors:
• **ZHENG, Hairong**
  **Shenzhen, Guangdong 518055 (CN)**

• **LIU, Xin**
  **Shenzhen, Guangdong 518055 (CN)**
• **ZOU, Chao**
  **Shenzhen, Guangdong 518055 (CN)**
• **CHENG, Chuanli**
  **Shenzhen, Guangdong 518055 (CN)**
• **QIAO, Yangzi**
  **Shenzhen, Guangdong 518055 (CN)**
• **TIE, Changjun**
  **Shenzhen, Guangdong 518055 (CN)**

(74) Representative: **de Arpe Fernandez, Manuel**
**Arpe Patentes y Marcas, S.L.P.**
**C/Proción, 7 Edif. América II**
**Portal 2, 1° C**
**28023 Madrid-Aravaca (ES)**

(54) **MAGNETIC RESONANCE TEMPERATURE IMAGING METHOD AND DEVICE**

(57) The present invention provides a magnetic resonance temperature imaging method and apparatus, and relates to the field of magnetic resonance. According to the magnetic resonance temperature imaging method and apparatus, accuracy and precision of a water-fat tissue temperature image at a current moment are improved by using a two-step iterative temperature estimation algorithm, a magnetic resonance signal model includes multiple fat peaks, and a fourth strength amplitude value of a water signal, a fourth strength amplitude value of a fat signal, a fourth field drift caused by a non-uniform main magnetic field, and the water-fat tissue temperature image at the current moment that minimize a difference between signal strength and signal strength before fitting are estimated.

S201 — Obtain a first strength amplitude value of a water signal, a first phase value of the water signal, a first strength amplitude value of a fat signal, a first phase value of the fat signal, a first transverse relaxation time of water, a first transverse relaxation time of fat, and a first field drift caused by a non-uniform main magnetic field, based on a preset magnetic resonance signal model, a pre-assigned initial water-fat tissue temperature image, and a water-fat separation algorithm

S202 — Obtain a second strength amplitude value of the water signal, a second phase value of the water signal, a second strength amplitude value of the fat signal, a second phase value of the fat signal, a second transverse relaxation time of the water, a second transverse relaxation time of the fat, and a second field drift caused by the non-uniform main magnetic field that minimize a difference between signal strength after the first time of fitting and signal strength before the fitting, by fitting the preset magnetic resonance signal model for the first time based on the initial water-fat tissue temperature image, the first strength amplitude value of the water signal, the first phase value of the water signal, the first strength amplitude value of the fat signal, the first phase value of the fat signal, the first transverse relaxation time of the water, the first transverse relaxation time of the fat, and the first field drift caused by the non-uniform main magnetic field

S203 — Smooth a second water-fat tissue temperature image by using a low-pass filter, to obtain a smoothed second water-fat tissue temperature image

S204 — Obtain a third strength amplitude value of the water signal, a third phase value of the water signal, a third strength amplitude value of the fat signal, a third phase value of the fat signal, a third transverse relaxation time of the water, a third transverse relaxation time of the fat, and a third field drift caused by the non-uniform main magnetic field that minimize a difference between signal strength after the second time of fitting and signal strength before the fitting, by fitting the preset magnetic resonance signal model by keeping the smoothed second water-fat tissue temperature image unchanged and based on the second water-fat tissue temperature image, the second strength amplitude value of the water signal, the second phase value of the water signal, the second strength amplitude value of the fat signal, the second phase value of the fat signal, the second transverse relaxation time of the water, the second transverse relaxation time of the fat, and the second field drift caused by the non-uniform main magnetic field

S205 — Obtain a fourth strength amplitude value of the water signal, a fourth phase value of the water signal, a fourth strength amplitude value of the fat signal, a fourth phase value of the fat signal, a fourth transverse relaxation time of the water, a fourth transverse relaxation time of the fat, a fourth field drift caused by the non-uniform main magnetic field that minimize a difference between signal strength after the third time of fitting and signal strength before the fitting, by fitting the preset magnetic resonance signal model for the third time by keeping the third phase value of the water signal, the third phase value of the fat signal, the third transverse relaxation time of the water, and the third transverse relaxation time of the fat unchanged and based on the second water-fat tissue temperature image, the third strength amplitude value of the water signal, the third phase value of the water signal, the third strength amplitude value of the fat signal, the third phase value of the fat signal, the third transverse relaxation time of the water, the third transverse relaxation time of the fat, and the third field drift caused by the non-uniform main magnetic field

FIG.2

**Description**

**CROSS-REFERENCE TO RELATED APPLICATIONS**

This application claims priority to Chinese Patent Application No. 201711387804.1, filed on December 20, 2017. The disclosures of the aforementioned patent applications are hereby incorporated by reference in their entireties.

**TECHNICAL FIELD**

The present invention relates to the field of magnetic resonance, and specifically, to a magnetic resonance temperature imaging method and apparatus.

**BACKGROUND**

Magnetic resonance temperature imaging can be used to monitor temperature distribution and changes of a tested object in a non-invasive, real time, and in vivo manner. Magnetic resonance can be used to monitor temperatures based on different temperature sensitive parameters, and common parameters include proton density (PD), a relaxation time, a diffusion coefficient, and a proton resonance frequency shift (PRFS). In water, there is a linear relationship between a proton resonance frequency and a temperature, and the linear relationship is tissue-independent, and therefore a PRFS-based magnetic resonance temperature imaging technology is most widely applied. However, this technology cannot be applied to fat-containing tissue because hydrogen protons in fat are temperature-insensitive. In this case, there is a non-linear relationship between the fat-containing tissue and a temperature change, and the non-linear relationship is related to a ratio of water to the fat in the tissue. To implement temperature imaging for the fat-containing tissue, impact of the fat needs to be eliminated or corrected.

**SUMMARY**

In view of this, embodiments of the present invention are intended to provide a magnetic resonance temperature imaging method and apparatus, to alleviate the foregoing problem.

According to a first aspect, an embodiment of the present invention provides a magnetic resonance temperature imaging method, where the magnetic resonance temperature imaging method includes:

Obtaining a first strength amplitude value of a water signal, a first phase value of the water signal, a first strength amplitude value of a fat signal, a first phase value of the fat signal, a first transverse relaxation time of water, a first transverse relaxation time of fat, and a first field drift caused by a non-uniform main magnetic field, based on a preset magnetic resonance signal model, a pre-assigned initial water-fat tissue temperature image, and a water-fat separation algorithm; obtaining a second strength amplitude value of the water signal, a second phase value of the water signal, a second strength amplitude value of the fat signal, a second phase value of the fat signal, a second transverse relaxation time of the water, a second transverse relaxation time of the fat, a second field drift caused by the non-uniform main magnetic field, and a second water-fat tissue temperature image that minimize a difference between signal strength after the first time of fitting and signal strength before the fitting, by fitting the preset magnetic resonance signal model for the first time based on the initial water-fat tissue temperature image, the first strength amplitude value of the water signal, the first phase value of the water signal, the first strength amplitude value of the fat signal, the first phase value of the fat signal, the first transverse relaxation time of the water, the first transverse relaxation time of the fat, and the first field drift caused by the non-uniform main magnetic field;

Obtaining a third strength amplitude value of the water signal, a third phase value of the water signal, a third strength amplitude value of the fat signal, a third phase value of the fat signal, a third transverse relaxation time of the water, a third transverse relaxation time of the fat, and a third field drift caused by the non-uniform main magnetic field that minimize a difference between signal strength after the second time of fitting and signal strength before the fitting, by fitting the preset magnetic resonance signal model for the second time by keeping the second water-fat tissue temperature image unchanged and based on the second water-fat tissue temperature image, the second strength amplitude value of the water signal, the second phase value of the water signal, the second strength amplitude value of the fat signal, the second phase value of the fat signal, the second transverse relaxation time of the water, the second transverse relaxation time of the fat, and the second field drift caused by the non-uniform main magnetic field; and

Obtaining a fourth strength amplitude value of the water signal, a fourth strength amplitude value of the fat signal, a fourth field drift caused by the non-uniform main magnetic field, and a water-fat tissue temperature image at a current moment that minimize a difference between signal strength after the third time of fitting and signal strength before the fitting, by fitting the preset magnetic resonance signal model for the third time by keeping the third phase value of the water signal, the third phase value of the fat signal, the third transverse relaxation time of the water, and the third transverse relaxation time of the fat unchanged and based on the second water-fat tissue temperature image, the third strength amplitude value of the water signal, the third phase value of the water signal, the third strength amplitude value of the fat signal, the third phase value of the fat signal, the third transverse relaxation time of the water, the third transverse relaxation time of the fat, and the third field drift caused by the non-uniform main magnetic field.

According to a second aspect, an embodiment of the present invention further provides a magnetic resonance temperature imaging apparatus, where the magnetic resonance temperature imaging apparatus includes:

A first calculation unit, configured to obtain a first strength amplitude value of a water signal, a first phase value of the water signal, a first strength amplitude value of a fat signal, a first phase value of the fat signal, a first transverse relaxation time of water, a first transverse relaxation time of fat, and a first field drift caused by a non-uniform main magnetic field, based on a preset magnetic resonance signal model, a pre-assigned initial water-fat tissue temperature image, and a water-fat separation algorithm;

A second calculation unit, configured to obtain a second strength amplitude value of the water signal, a second phase value of the water signal, a second strength amplitude value of the fat signal, a second phase value of the fat signal, a second transverse relaxation time of the water, a second transverse relaxation time of the fat, a second field drift caused by the non-uniform main magnetic field, and a second water-fat tissue temperature image that minimize a difference between signal strength after the first time of fitting and signal strength before the fitting, by fitting the preset magnetic resonance signal model for the first time based on the initial water-fat tissue temperature image, the first strength amplitude value of the water signal, the first phase value of the water signal, the first strength amplitude value of the fat signal, the first phase value of the fat signal, the first transverse relaxation time of the water, the first transverse relaxation time of the fat, and the first field drift caused by the non-uniform main magnetic field;

A third calculation unit, configured to obtain a third strength amplitude value of the water signal, a third phase value of the water signal, a third strength amplitude value of the fat signal, a third phase value of the fat signal, a third transverse relaxation time of the water, a third transverse relaxation time of the fat, and a third field drift caused by the non-uniform main magnetic field that minimize a difference between signal strength after the second time of fitting and signal strength before the fitting, by fitting the preset magnetic resonance signal model for the second time by keeping the second water-fat tissue temperature image unchanged and based on the second water-fat tissue temperature image, the second strength amplitude value of the water signal, the second phase value of the water signal, the second strength amplitude value of the fat signal, the second phase value of the fat signal, the second transverse relaxation time of the water, the second transverse relaxation time of the fat, and the second field drift caused by the non-uniform main magnetic field; and

A fourth calculation unit, configured to obtain a fourth strength amplitude value of the water signal, a fourth strength amplitude value of the fat signal, a fourth field drift caused by the non-uniform main magnetic field, and a water-fat tissue temperature image at a current moment that minimize a difference between signal strength after the third time of fitting and signal strength before the fitting, by fitting the preset magnetic resonance signal model for the third time by keeping the third phase value of the water signal, the third phase value of the fat signal, the third transverse relaxation time of the water, and the third transverse relaxation time of the fat unchanged and based on the second water-fat tissue temperature image, the third strength amplitude value of the water signal, the third phase value of the water signal, the third strength amplitude value of the fat signal, the third phase value of the fat signal, the third transverse relaxation time of the water, the third transverse relaxation time of the fat, and the third field drift caused by the non-uniform main magnetic field.

According to the magnetic resonance temperature imaging method and apparatus provided in the present invention, accuracy and precision of the water-fat tissue temperature image at the current moment are improved by using a two-step iterative temperature estimation algorithm in comparison with the prior art, the magnetic resonance signal model includes multiple fat peaks, and the fourth strength amplitude value of the water signal, the fourth strength amplitude value of the fat signal, the fourth field drift caused by the non-uniform main magnetic field, and the water-fat tissue temperature image at the current moment that minimize the difference between the signal strength and the signal strength before the fitting are estimated. In this way, it is ensured that a temperature result is unbiased.

To make the foregoing objectives, features, and advantages of the present invention clearer and easier to understand, preferred embodiments are described in detail below with reference to the accompanying drawings.

**BRIEF DESCRIPTION OF DRAWINGS**

FIG. 1 is a structural block diagram of a server according to the present invention;
FIG. 2 is a flowchart of a magnetic resonance temperature imaging method according to the present invention; and
FIG. 3 is a functional and structural block diagram of a magnetic resonance temperature imaging apparatus according to the present invention.

Reference numerals: 100-Magnetic resonance temperature imaging apparatus; 200-Server; 101-Memory; 102-Storage controller; 103-Processor; 104-Peripheral interface; 301-First calculation unit; 302-Second calculation unit; 303-Third calculation unit; 304-Filtering unit; 305-Fourth calculation unit.

**DESCRIPTION OF EMBODIMENTS**

The following clearly and comprehensively describes the technical solutions in the embodiments of the present invention with reference to the accompanying drawings in the embodiments of the present invention. Clearly, the described embodiments are merely some but not all of the embodiments of the present invention. Components in the embodiments of the present invention described and shown in the accompanying drawings usually can be arranged and designed in various different configurations. Therefore, the following detailed description of the embodiments of the present invention provided in the accompanying drawings is not intended to limit the protection scope of the present invention, but is merely intended to represent the selected embodiments of the present invention. All other embodiments obtained by a person skilled in the art based on the embodiments of the present invention without creative efforts shall fall within the protection scope of the present invention.

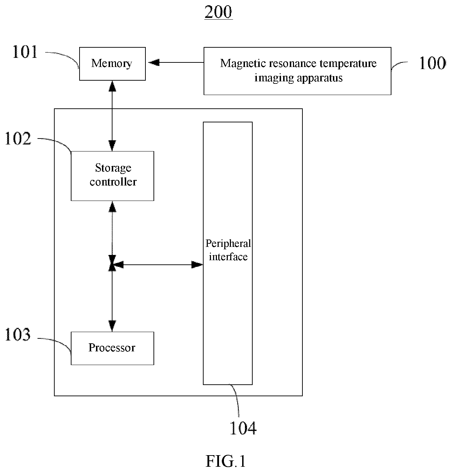

A magnetic resonance temperature imaging method and apparatus provided in the preferred embodiments of the present invention can be applied to a server 200. FIG. 1 is a structural block diagram of a server 200 according to an embodiment of the present invention. As shown in FIG. 1, the server 200 includes a magnetic resonance temperature imaging apparatus 100, a memory 101, a storage controller 102, one or more processors (only one processor is shown in the figure) 103, a peripheral interface 104, and the like. These components communicate with each other by using one or more communications buses/signal lines. The magnetic resonance temperature imaging apparatus 100 includes at least one software function module that may be stored in the memory 101 in the form of software or firmware or that may be built into an operating system (OS) of the server 200.

The memory 101 may be configured to store a software program and a module, for example, a program instruction/module corresponding to the magnetic resonance temperature image apparatus and method in the embodiments of the present invention. The processor 103 executes various functional applications and data processing, for example, the magnetic resonance temperature imaging method provided in the embodiments of the present invention, by running the software program and the module stored in the memory 101. The memory 101 may include a high speed random access memory, and may further include a nonvolatile memory, for example, one or more magnetic storage apparatuses or flash memories, or other nonvolatile solid state memories. Access to the memory 101 by the processor 103 and other possible components may be under the control of the storage controller 102.

The peripheral interface 104 couples various input/output apparatuses to the processor 103 and the memory 101. In some embodiments, the peripheral interface 104, the processor 103, and the storage controller 102 may be implemented in a single chip. In other examples, the peripheral interface 104, the processor 103, and the storage controller 102 each may be implemented by a separate chip.

It can be understood that the structure shown in FIG. 1 is merely an example, and the server 200 may alternatively include more or fewer components than those shown in FIG. 1, or have a configuration different from that shown in FIG. 1. The components shown in FIG. 1 may be implemented by hardware, software, or a combination thereof.

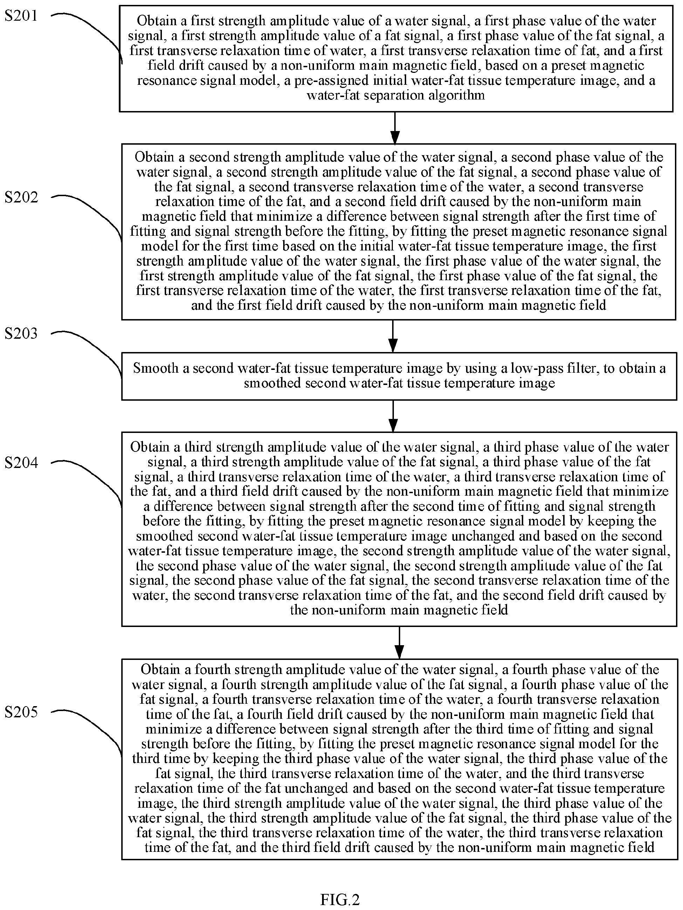

Referring to FIG. 2, an embodiment of the present invention provides a magnetic resonance temperature imaging method. The magnetic resonance temperature imaging method includes the following steps.

Step S201: Obtain a first strength amplitude value of a water signal, a first phase value of the water signal, a first strength amplitude value of a fat signal, a first phase value of the fat signal, a first transverse relaxation time of water, a first transverse relaxation time of fat, and a first field drift caused by a non-uniform main magnetic field, based on a preset magnetic resonance signal model, a pre-assigned initial water-fat tissue temperature image, and a water-fat separation algorithm.

Specifically, the preset magnetic resonance signal model is

$$s_n = \left( W e^{-i2\pi\gamma B_0 \alpha TE_n \Delta T} e^{-\frac{TE_n}{T_{2,W}^*}} + F \sum_{p=1}^{P} \beta_p e^{-i2\pi\gamma B_0 f_{F,p} TE_n} e^{-\frac{TE_n}{T_{2,F}^*}} \right) e^{-i2\pi f_B TE_n}, n = 1, 2, ..., N$$

, where $S_n$ represents signal strength at an echo time $TE_n$, $W$ represents a strength value of the water signal, $F$ represents a strength value of the fat signal, $Y$ represents a gyromagnetic ratio, $B_0$ represents strength of the main magnetic field, $a$ represents a temperature coefficient of a hydrogen proton in the water, P represents the number of fat peaks, a corresponding relative amplitude value and chemical shift are respectively $\beta_p$ and $f_{F,p}$, $\sum_{p=1}^{p} \beta_p = 1$, $T_{2,W}^*$ represents a transverse relaxation time of the water, $T_{2,F}^{*}$ represents a transverse relaxation time of the fat, fb represents a field drift caused by the non-uniform main magnetic field, N represents the total number of collected echoes, and $\Delta T$ represents a water-fat tissue temperature image.

Step S202: Obtain a second strength amplitude value of the water signal, a second phase value of the water signal, a second strength amplitude value of the fat signal, a second phase value of the fat signal, a second transverse relaxation time of the water, a second transverse relaxation time of the fat, a second field drift caused by the non-uniform main magnetic field, and a second water-fat tissue temperature image that minimize a difference between signal strength after the first time of fitting and signal strength before the fitting, by fitting the preset magnetic resonance signal model for the first time based on the initial water-fat tissue temperature image, the first strength amplitude value of the water signal, the first phase value of the water signal, the first strength amplitude value of the fat signal, the first phase value of the fat signal, the first transverse relaxation time of the water, the first transverse relaxation time of the fat, and the first field drift caused by the non-uniform main magnetic field.

Specifically, the preset magnetic resonance signal model is fitted for the first time based on an equation

$$\left\{ A_{W}, \Phi_{W}, T_{2,W}^{*}, A_{F}, \Phi_{F}, T_{2,F}^{*}, f_{B}, \Delta T \right\} =$$

$$\arg\min \sum_{n=1}^{N} \left( s_{n} - \left( We^{-i2\pi\gamma B_{0}\alpha TE_{n}\Delta T} e^{-\frac{TE_{n}}{T_{2,W}^{*}}} + F\sum_{p=1}^{P} \beta_{p} e^{-i2\pi\gamma B_{0}f_{F,p}TE_{n}} e^{-\frac{TE_{n}}{T_{2,F}^{*}}} \right) e^{-i2\pi f_{B}TE_{n}} \right)^{2}.$$

Step S203: Smooth the second water-fat tissue temperature image by using a low-pass filter, to obtain a smoothed second water-fat tissue temperature image.

Specifically, the second water-fat tissue temperature image is smoothed by using the low-pass filter based on an equation $\Delta \tilde{T}_{i} = (1-\mu)\Delta T_{i} + \mu \Delta \overline{T}$, to obtain the smoothed second water-fat tissue temperature image, where $\Delta T_{i}$ represents a current temperature estimation value of the $i^{\text{th}}$ pixel, $\Delta \overline{T}$ represents an average value of temperature values of all pixels, and $\Delta \tilde{T}_{i}$ represents a temperature value obtained after the $i^{\text{th}}$ pixel is smoothed.

Step S204: Obtain a third strength amplitude value of the water signal, a third phase value of the water signal, a third strength amplitude value of the fat signal, a third phase value of the fat signal, a third transverse relaxation time of the water, a third transverse relaxation time of the fat, and a third field drift caused by the non-uniform main magnetic field that minimize a difference between signal strength after the second time of fitting and signal strength before the fitting, by fitting the preset magnetic resonance signal model for the second time by keeping the smoothed second water-fat tissue temperature image unchanged and based on the second water-fat tissue temperature image, the second strength amplitude value of the water signal, the second phase value of the water signal, the second strength amplitude value of the fat signal, the second phase value of the fat signal, the second transverse relaxation time of the water, the second transverse relaxation time of the fat, and the second field drift caused by the non-uniform main magnetic field.

Specifically, the preset magnetic resonance signal model is fitted for the second time based on an equation

$$\left\{ A_{W}, \Phi_{W}, T_{2,W}^{*}, A_{F}, \Phi_{F}, T_{2,F}^{*}, f_{B} \right\} =$$

$$\arg\min \sum_{n=1}^{N} \left( s_{n} - \left( We^{-i2\pi\gamma B_{0}\alpha TE_{n}\Delta \tilde{T}} e^{-\frac{TE_{n}}{T_{2,W}^{*}}} + F\sum_{p=1}^{P} \beta_{p} e^{-i2\pi\gamma B_{0}f_{F,p}TE_{n}} e^{-\frac{TE_{n}}{T_{2,F}^{*}}} \right) e^{-i2\pi f_{B}TE_{n}} \right)^{2}.$$

Step S205: Obtain a fourth strength amplitude value of the water signal, a fourth strength amplitude value of the fat signal, a fourth field drift caused by the non-uniform main magnetic field, and a water-fat tissue temperature image at a current moment that minimize a difference between signal strength after the third time of fitting and signal strength before the fitting, by fitting the preset magnetic resonance signal model for the third time by keeping the third phase value of the water signal, the third phase value of the fat signal, the third transverse relaxation time of the water, and the third transverse relaxation time of the fat unchanged and based on the second water-fat tissue temperature image, the third strength amplitude value of the water signal, the third phase value of the water signal, the third strength amplitude value of the fat signal, the third phase value of the fat signal, the third transverse relaxation time of the water, the third transverse relaxation time of the fat, and the third field drift caused by the non-uniform main magnetic field.

Specifically, the preset magnetic resonance signal model is fitted for the third time based on an equation

$$\{A_W, A_F, f_B, \Delta T\} =$$

$$\arg\min \sum_{n=1}^{N} \left( s_n - \left( A_W e^{i\phi_W} e^{-i2\pi\gamma B_0 \alpha TE_n \Delta T} e^{-\frac{TE_n}{T_{2,W}^*}} + A_F e^{i\phi_F} \sum_{p=1}^{P} \beta_p e^{-i2\pi\gamma B_0 f_{F,p} TE_n} e^{-\frac{TE_n}{T_{2,F}^*}} \right) e^{-i2\pi f_B TE_n} \right)^2$$

According to the magnetic resonance temperature imaging method, first, the preset magnetic resonance signal model is fitted to obtain the second strength amplitude value of the water signal, the second phase value of the water signal, the second strength amplitude value of the water signal, the second phase value of the water signal, the second transverse relaxation time of the water, the second transverse relaxation time of the fat, the second field drift caused by the non-uniform main magnetic field, and the second water-fat tissue temperature image that minimize the difference between the signal strength and the signal strength before the fitting, to ensure that temperature estimation is unbiased, in other words, ensure accuracy of the second water-fat tissue temperature image; then, the second water-fat tissue temperature image is smoothed, and the third strength amplitude value of the water signal, the third phase value of the water signal, the third strength amplitude value of the fat signal, the third phase value of the fat signal, the third transverse relaxation time of the water, the third transverse relaxation time of the fat, and the third field drift caused by the non-uniform main magnetic field that minimize the difference between the signal strength after the second time of fitting and the signal strength before the fitting are estimated, where in this case, the obtained third phase value of the water signal, third phase value of the fat signal, third transverse relaxation time of the water, and third transverse relaxation time of the fat are accurate; and finally, the fourth strength amplitude value of the water signal, the fourth strength amplitude value of the fat signal, the fourth field drift caused by the non-uniform main magnetic field, and the water-fat tissue temperature image at the current moment are obtained by fitting the signal model again by keeping the third phase value of the water signal, the third phase value of the fat signal, the third transverse relaxation time of the water, and the third transverse relaxation time of the fat unchanged, where in this case, precision of the obtained preset magnetic resonance signal model is improved because of a decreased number of free variables in the preset magnetic resonance signal model.

In addition, the water-fat tissue temperature image at the current moment may be used as an initial value of the next cycle, and steps S201 to S205 are repeated to obtain a water-fat tissue temperature image at the next moment.

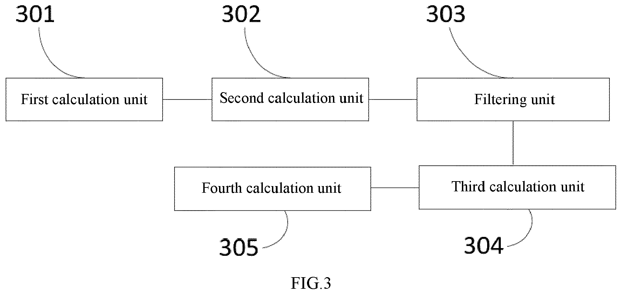

Referring to FIG. 3, an embodiment of the present invention further provides a magnetic resonance temperature imaging apparatus 100. It should be noted that basic principles and technical effects of the magnetic resonance temperature imaging apparatus 100 provided in this embodiment of the present invention are the same as those in the foregoing embodiment. For brevity, refer to the corresponding content in the foregoing embodiment for content not mentioned in this embodiment of the present invention. The magnetic resonance temperature imaging apparatus 100 includes a first calculation unit 301, a second calculation unit 302, a third calculation unit 303, a filtering unit 304, and a fourth calculation unit 305.

The first calculation unit 301 is configured to obtain a first strength amplitude value of a water signal, a first phase value of the water signal, a first strength amplitude value of a fat signal, a first phase value of the fat signal, a first transverse relaxation time of water, a first transverse relaxation time of fat, and a first field drift caused by a non-uniform main magnetic field, based on a preset magnetic resonance signal model, a pre-assigned initial water-fat tissue temperature image, and a water-fat separation algorithm.

The preset magnetic resonance signal model is

$$s_n = \left( W e^{-i2\pi\gamma B_0 \alpha TE_n \Delta T} e^{\frac{TE_n}{T_{2,W}^*}} + F \sum_{p=1}^{P} \beta_p e^{-i2\pi\gamma B_0 f_{F,p} TE_n} e^{\frac{TE_n}{T_{2,F}^*}} \right) e^{-i2\pi f_B TE_n}, n = 1, 2, ..., N$$

, where $S_n$ represents signal strength at an echo time $TE_n$, $W$ represents a strength value of the water signal, $F$ represents a strength value of the fat signal, $Y$ represents a gyromagnetic ratio, $B_0$ represents strength of the main magnetic field, $a$ represents a temperature coefficient of a hydrogen proton in the water, $P$ represents the number of fat peaks, a corresponding relative amplitude value and chemical shift are respectively $\beta_p$ and $f_{F,p}$, $\sum_{p=1}^{p} \beta_p = 1$, $T_{2,W}^*$ represents a transverse relaxation time of the water, $T_{2,F}^*$ represents a transverse relaxation time of the fat, fb represents a field drift caused by the non-uniform main magnetic field, N represents the total number of collected echoes, and $\Delta T$ represents a water-fat tissue temperature image.

It can be understood that the first calculation unit 301 can perform step S201.

The second calculation unit 302 is configured to obtain a second strength amplitude value of the water signal, a second phase value of the water signal, a second strength amplitude value of the water signal, a second phase value of the water signal, a second transverse relaxation time of the water, a second transverse relaxation time of the fat, a second field drift caused by the non-uniform main magnetic field, and a second water-fat tissue temperature image that minimize a difference between signal strength after the first time of fitting and signal strength before the fitting, by fitting the preset magnetic resonance signal model for the first time based on the initial water-fat tissue temperature image, the first strength amplitude value of the water signal, the first phase value of the water signal, the first strength amplitude value of the fat signal, the first phase value of the fat signal, the first transverse relaxation time of the water, the first transverse relaxation time of the fat, and the first field drift caused by the non-uniform main magnetic field.

The second calculation unit 302 is configured to fit the preset magnetic resonance signal model for the first time based on an equation

$$\left\{A_W, \Phi_W, T_{2,W}^*, A_F, \Phi_F, T_{2,F}^*, f_B, \Delta T\right\} =$$

$$\arg\min \sum_{n=1}^{N} \left( s_n - \left( W e^{-i2\pi\gamma B_0 \alpha T E_n \Delta T} e^{\frac{TE_n}{T_{2,W}^*}} + F \sum_{p=1}^{P} \beta_p e^{-i2\pi\gamma B_0 f_{F,p} T E_n} e^{\frac{TE_n}{T_{2,F}^*}} \right) e^{-i2\pi f_B T E_n} \right)^2.$$

It can be understood that the second calculation unit 302 can perform step S202.

The filtering unit 304 is configured to smooth the second water-fat tissue temperature image by using a low-pass filter, to obtain a smoothed second water-fat tissue temperature image.

It can be understood that the filtering unit 304 can perform step S203.

The filtering unit 304 is configured to smooth the second water-fat tissue temperature image by using the low-pass filter based on an equation $\Delta\tilde{T}_i = (1-\mu)\Delta T_i + \mu\Delta\overline{T}$, to obtain the smoothed second water-fat tissue temperature image, where $\Delta T_i$ represents a current temperature estimation value of the i$^{th}$ pixel, $\Delta\overline{T}$ represents an average value of temperature values of all pixels, and $\Delta\tilde{T}_i^{''}$ represents a temperature value obtained after the i$^{th}$ pixel is smoothed.

The third calculation unit 303 is configured to obtain a third strength amplitude value of the water signal, a third phase value of the water signal, a third strength amplitude value of the fat signal, a third phase value of the fat signal, a third transverse relaxation time of the water, a third transverse relaxation time of the fat, and a third field drift caused by the non-uniform main magnetic field that minimize a difference between signal strength after the second time of fitting and signal strength before the fitting, by fitting the preset magnetic resonance signal model for the second time by keeping the second water-fat tissue temperature image unchanged and based on the second water-fat tissue temperature image, the second strength amplitude value of the water signal, the second phase value of the water signal, the second strength amplitude value of the fat signal, the second phase value of the fat signal, the second transverse relaxation time of the water, the second transverse relaxation time of the fat, and the second field drift caused by the non-uniform main magnetic field.

The third calculation unit 303 is configured to fit the preset magnetic resonance signal

$$\left\{A_W, \Phi_W, T_{2,W}^*, A_F, \Phi_F, T_{2,F}^*, f_B\right\} =$$

$$\arg\min \sum_{n=1}^{N} \left( s_n - \left( W e^{-i2\pi\gamma B_0 \alpha T E_n \Delta\tilde{T}} e^{\frac{TE_n}{T_{2,W}^*}} + F \sum_{p=1}^{P} \beta_p e^{-i2\pi\gamma B_0 f_{F,p} T E_n} e^{\frac{TE_n}{T_{2,F}^*}} \right) e^{-i2\pi f_B T E_n} \right)^2.$$

model for the second time based on an equation

It can be understood that the third calculation unit 303 can perform step S204.

The fourth calculation unit 305 is configured to obtain a fourth strength amplitude value of the water signal, a fourth strength amplitude value of the fat signal, a fourth field drift caused by the non-uniform main magnetic field, and a water-fat tissue temperature image at a current moment that minimize a difference between signal strength after the third time of fitting and signal strength before the fitting, by fitting the preset magnetic resonance signal model for the third time by keeping the third phase value of the water signal, the third phase value of the fat signal, the third transverse relaxation time of the water, and the third transverse relaxation time of the fat unchanged and based on the second water-fat tissue temperature image, the third strength amplitude value of the water signal, the third phase value of the water signal, the third strength amplitude value of the water signal, the third phase value of the water signal, the third transverse relaxation time of the water, the third transverse relaxation time of the fat, and the third field drift caused by the non-uniform main magnetic field.

Specifically, the fourth calculation unit 305 is configured to fit the preset magnetic resonance signal model for the third time based on an equation

$$\{A_W, A_F, f_B, \Delta T\} = \arg\min \sum_{n=1}^{N} \left( s_n - \left( A_W e^{i\tilde{\phi}_W} e^{-i2\pi\gamma B_0 \alpha T E_n \Delta T} e^{\frac{TE_n}{T_{2,W}^*}} + A_F e^{i\tilde{\phi}_F} \sum_{p=1}^{P} \beta_p e^{-i2\pi\gamma B_0 f_{F,p} TE_n} e^{\frac{TE_n}{T_{2,F}^*}} \right) e^{-i2\pi f_B TE_n} \right)^2 .$$

It can be understood that the fourth calculation unit 305 can perform step S205.

In conclusion, according to the magnetic resonance temperature imaging method and apparatus, accuracy and precision of the water-fat tissue temperature image at the current moment are improved by using a two-step iterative temperature estimation algorithm, the magnetic resonance signal model includes multiple fat peaks, and the fourth strength amplitude value of the water signal, the fourth strength amplitude value of the fat signal, the fourth field drift caused by the non-uniform main magnetic field, and the water-fat tissue temperature image at the current moment that minimize the difference between the signal strength and the signal strength before the fitting are estimated. In this way, it is ensured that a temperature result is unbiased.

In the several embodiments provided in this application, it should be understood that the disclosed apparatus and method may be implemented in other manners. The apparatus embodiment described above is merely an example. For example, flowcharts and block diagrams in the accompanying drawings show possible implementation architectures, functions, and operations of the apparatus, the method, and the computer program product according to the multiple embodiments of the present invention. In this regard, each block in the flowchart or block diagram may represent a part of one module, program segment, or code. The part of the module, program segment, or code includes one or more executable instructions for implementing a specified logical function. It should also be noted that in some alternative implementations, the functions noted in the blocks may be executed in an order different from that noted in the accompanying drawings. For example, two consecutive blocks may actually be executed substantially in parallel, or two consecutive blocks may sometimes be executed in a reverse order, and this depends on functions involved. It should also be noted that each block in the block diagrams and/or flowcharts and a combination of blocks in the block diagrams and/or flowcharts may be implemented by using a dedicated hardware-based system for executing a specified function or action, or may be implemented by using a combination of dedicated hardware and a computer instruction.

In addition, the functional modules in the various embodiments of the present invention may be integrated together to form a separate part, or each module may exist alone, or two or more modules may be integrated to form a separate part.

If the functions are implemented in the form of software functional modules and sold or used as separate products, the functions may be stored in a computer-readable storage medium. Based on such an understanding, the technical solutions of the present invention, essentially or the part contributing to the prior art, may be embodied in the form of a software product. The computer software product is stored in a storage medium, and includes several instructions for enabling a computer device (which may be a personal computer, a server, a network device, or the like) to perform all or some of the steps of the method described in the embodiments of the present invention. The storage medium includes various media that can store program code, for example, a USB flash drive, a removable hard disk, a read-only memory (ROM), a random access memory (RAM), a magnetic disk, and an optical disk. It should be noted that in this specification, relational terms such as first and second are used only to distinguish one entity or operation from another entity or operation, without necessarily requiring or implying any such actual relationship or order between such entities or operations. In addition, the term "include", "comprise", or their any other variant is intended to cover a non-exclusive inclusion, so that a process, a method, an article, or a device that includes a series of elements not only includes these elements, but also includes other elements that are not expressly listed, or further includes elements inherent to such process, method, article, or device. An element preceded by "includes a ..." further includes, without more constraints, additional identical elements in the process, method, article, or device that includes the element.

The foregoing descriptions are merely preferred embodiments of the present invention, and are not intended to limit the present invention. A person skilled in the art can make various modifications and changes to the present invention. Any modifications, equivalent replacements, and improvements made within the spirit and the principle of the present invention shall fall within the protection scope of the present invention. It should be noted that similar reference numerals and letters represent similar terms in the following accompanying drawings. Therefore, once an item is defined in one accompanying drawing, the item does not need to be further defined and explained in subsequent accompanying drawings.

The foregoing descriptions are merely specific implementations of the present invention, but the protection scope of the present invention is not limited thereto. Variations or replacements that can be readily figured out by any person skilled in the art within the technical scope of the present invention shall fall within the protection scope of the present invention. Therefore, the protection scope of the present invention should be subject to the protection scope of the claims.

It should be noted that in this specification, relational terms such as first and second are used only to distinguish one entity or operation from another entity or operation, without necessarily requiring or implying any such actual relationship or order between such entities or operations. In addition, the term "include", "comprise", or their any other variant is intended to cover a non-exclusive inclusion, so that a process, a method, an article, or a device that includes a series of elements not only includes these elements, but also includes other elements that are not expressly listed, or further includes elements inherent to such process, method, article, or device. An element preceded by "includes a ..." further includes, without more constraints, additional identical elements in the process, method, article, or device that includes the element.

**Claims**

1. A magnetic resonance temperature imaging method, wherein the magnetic resonance temperature imaging method comprises:

obtaining a first strength amplitude value of a water signal, a first phase value of the water signal, a first strength amplitude value of a fat signal, a first phase value of the fat signal, a first transverse relaxation time of water, a first transverse relaxation time of fat, and a first field drift caused by a non-uniform main magnetic field, based on a preset magnetic resonance signal model, a pre-assigned initial water-fat tissue temperature image, and a water-fat separation algorithm;

obtaining a second strength amplitude value of the water signal, a second phase value of the water signal, a second strength amplitude value of the fat signal, a second phase value of the fat signal, a second transverse relaxation time of the water, a second transverse relaxation time of the fat, a second field drift caused by the non-uniform main magnetic field, and a second water-fat tissue temperature image that minimize a difference between signal strength after the first time of fitting and signal strength before the fitting, by fitting the preset magnetic resonance signal model for the first time based on the initial water-fat tissue temperature image, the first strength amplitude value of the water signal, the first phase value of the water signal, the first strength amplitude value of the fat signal, the first phase value of the fat signal, the first transverse relaxation time of the water, the first transverse relaxation time of the fat, and the first field drift caused by the non-uniform main magnetic field;

obtaining a third strength amplitude value of the water signal, a third phase value of the water signal, a third strength amplitude value of the fat signal, a third phase value of the fat signal, a third transverse relaxation time of the water, a third transverse relaxation time of the fat, and a third field drift caused by the non-uniform main magnetic field that minimize a difference between signal strength after the second time of fitting and signal strength before the fitting, by fitting the preset magnetic resonance signal model for the second time by keeping the second water-fat tissue temperature image unchanged and based on the second water-fat tissue temperature image, the second strength amplitude value of the water signal, the second phase value of the water signal, the second strength amplitude value of the fat signal, the second phase value of the fat signal, the second transverse relaxation time of the water, the second transverse relaxation time of the fat, and the second field drift caused by the non-uniform main magnetic field; and

obtaining a fourth strength amplitude value of the water signal, a fourth strength amplitude value of the fat signal, a fourth field drift caused by the non-uniform main magnetic field, and a water-fat tissue temperature image at a current moment that minimize a difference between signal strength after the third time of fitting and signal strength before the fitting, by fitting the preset magnetic resonance signal model for the third time by keeping the third phase value of the water signal, the third phase value of the fat signal, the third transverse relaxation time of the water, and the third transverse relaxation time of the fat unchanged and based on the second water-fat tissue temperature image, the third strength amplitude value of the water signal, the third phase value of the water signal, the third strength amplitude value of the fat signal, the third phase value of the fat signal, the third transverse relaxation time of the water, the third transverse relaxation time of the fat, and the third field drift caused by the non-uniform main magnetic field.

2. The magnetic resonance temperature imaging method according to claim 1, wherein before the step of obtaining a third strength amplitude value of the water signal, a third phase value of the water signal, a third strength amplitude value of the fat signal, a third phase value of the fat signal, a third transverse relaxation time of the water, a third transverse relaxation time of the fat, and a third field drift caused by the non-uniform main magnetic field that minimize a difference between signal strength after the second time of fitting and signal strength before the fitting, by fitting the preset magnetic resonance signal model for the second time by keeping the second water-fat tissue temperature image unchanged and based on the second water-fat tissue temperature image, the second strength amplitude value of the water signal, the second phase value of the water signal, the second strength amplitude value of the fat signal, the second phase value of the fat signal, the second transverse relaxation time of the water, the second transverse relaxation time of the fat, and the second field drift caused by the non-uniform main magnetic field, the magnetic resonance temperature imaging method comprises:
smoothing the second water-fat tissue temperature image by using a low-pass filter, to obtain a smoothed second water-fat tissue temperature image.

3. The magnetic resonance temperature imaging method according to claim 2, wherein the second water-fat tissue temperature image is smoothed by using the low-pass filter based on an equation $\Delta\tilde{T}_i = (1-\mu)\Delta T_i + \mu\Delta\overline{T}$, to obtain the smoothed second water-fat tissue temperature image, wherein $\Delta T_i$ represents a current temperature estimation value of the $i^{th}$ pixel, $\Delta\overline{T}$ represents an average value of temperature values of all pixels, and $\Delta\tilde{T}_i$ represents a temperature value obtained after the $i^{th}$ pixel is smoothed.

4. The magnetic resonance temperature imaging method according to claim 1, wherein the preset magnetic resonance signal model is

$$S_n = \left(We^{-i2\pi\gamma B_0 \alpha TE_n \Delta T} e^{\frac{TE_n}{T_{2,W}^*}} + F\sum_{p=1}^{P}\beta_p e^{-i2\pi\gamma B_0 f_{F,p} TE_n} e^{\frac{TE_n}{T_{2,F}^*}}\right)e^{-i2\pi f_B TE_n}, n = 1, 2, ..., N$$

wherein $S_n$ represents signal strength at an echo time $TE_n$, $W$ represents a strength value of the water signal, F represents a strength value of the fat signal, $Y$ represents a gyromagnetic ratio, $B_0$ represents strength of the main magnetic field, $a$ represents a temperature coefficient of a hydrogen proton in the water, P represents the number of fat peaks, a corresponding relative amplitude value and chemical shift are respectively $\beta_p$ and $f_{F,p}$,

$\sum_{p=1}^{p}\beta_p = 1$ represents a transverse relaxation time of the water, $T_{2,F}^*$ represents a transverse relaxation time of the fat, fb represents a field drift caused by the non-uniform main magnetic field, N represents the total number of collected echoes, and $\Delta T$ represents a water-fat tissue temperature image.

5. The magnetic resonance temperature imaging method according to claim 4, wherein the preset magnetic resonance signal model is fitted for the first time based on an equation

$$\left\{A_W, \Phi_W, T_{2,W}^*, A_F, \Phi_F, T_{2,F}^*, f_B, \Delta T\right\} =$$

$$\arg\min\sum_{n=1}^{N}\left(S_n - \left(We^{-i2\pi\gamma B_0\alpha TE_n\Delta T}e^{\frac{TE_n}{T_{2,W}^*}} + F\sum_{p=1}^{P}\beta_p e^{-i2\pi\gamma B_0 f_{F,p} TE_n}e^{\frac{TE_n}{T_{2,F}^*}}\right)e^{-i2\pi f_B TE_n}\right)^2.$$

6. A magnetic resonance temperature imaging apparatus, wherein the magnetic resonance temperature imaging apparatus comprises:

a first calculation unit, configured to obtain a first strength amplitude value of a water signal, a first phase value of the water signal, a first strength amplitude value of a fat signal, a first phase value of the fat signal, a first transverse relaxation time of water, a first transverse relaxation time of fat, and a first field drift caused by a non-uniform main magnetic field, based on a preset magnetic resonance signal model, a pre-assigned initial water-fat tissue temperature image, and a water-fat separation algorithm;

a second calculation unit, configured to obtain a second strength amplitude value of the water signal, a second phase value of the water signal, a second strength amplitude value of the fat signal, a second phase value of the fat signal, a second transverse relaxation time of the water, a second transverse relaxation time of the fat, a second field drift caused by the non-uniform main magnetic field, and a second water-fat tissue temperature image that minimize a difference between signal strength after the first time of fitting and signal strength before the fitting, by fitting the preset magnetic resonance signal model for the first time based on the initial water-fat tissue temperature image, the first strength amplitude value of the water signal, the first phase value of the water signal, the first strength amplitude value of the fat signal, the first phase value of the fat signal, the first transverse relaxation time of the water, the first transverse relaxation time of the fat, and the first field drift caused by the non-uniform main magnetic field;

a third calculation unit, configured to obtain a third strength amplitude value of the water signal, a third phase value of the water signal, a third strength amplitude value of the fat signal, a third phase value of the fat signal, a third transverse relaxation time of the water, a third transverse relaxation time of the fat, and a third field drift caused by the non-uniform main magnetic field that minimize a difference between signal strength after the second time of fitting and signal strength before the fitting, by fitting the preset magnetic resonance signal model for the second time by keeping the second water-fat tissue temperature image unchanged and based on the second water-fat tissue temperature image, the second strength amplitude value of the water signal, the second phase value of the water signal, the second strength amplitude value of the fat signal, the second phase value of the fat signal, the second transverse relaxation time of the water, the second transverse relaxation time of the fat, and the second field drift caused by the non-uniform main magnetic field; and

a fourth calculation unit, configured to obtain a fourth strength amplitude value of the water signal, a fourth strength amplitude value of the fat signal, a fourth field drift caused by the non-uniform main magnetic field, and a water-fat tissue temperature image at a current moment that minimize a difference between signal strength after the third time of fitting and signal strength before the fitting, by fitting the preset magnetic resonance signal model for the third time by keeping the third phase value of the water signal, the third phase value of the fat signal, the third transverse relaxation time of the water, and the third transverse relaxation time of the fat unchanged and based on the second water-fat tissue temperature image, the third strength amplitude value of the water signal, the third phase value of the water signal, the third strength amplitude value of the fat signal, the third phase value of the fat signal, the third transverse relaxation time of the water, the third transverse relaxation time of the fat, and the third field drift caused by the non-uniform main magnetic field.

7. The magnetic resonance temperature imaging apparatus according to claim 6, wherein the magnetic resonance temperature imaging apparatus further comprises:
a filtering unit, configured to smooth the second water-fat tissue temperature image by using a low-pass filter, to obtain a smoothed second water-fat tissue temperature image.

8. The magnetic resonance temperature imaging apparatus according to claim 7, wherein the filtering unit is configured to smooth the second water-fat tissue temperature image by using the low-pass filter based on an equation $\Delta \tilde{T}_i=(1-\mu)\Delta T_i+\mu \Delta \overline{T}$, to obtain the smoothed second water-fat tissue temperature image, wherein $\Delta T_i$ represents a current temperature estimation value of the $i^{th}$ pixel, $\Delta \overline{T}$ represents an average value of temperature values of all pixels, and $\Delta \tilde{T}_i$ represents a temperature value obtained after the $i^{th}$ pixel is smoothed.

9. The magnetic resonance temperature imaging apparatus according to claim 6, wherein the preset magnetic resonance signal model is

$$s_n = \left( We^{-i2\pi\gamma B_0 \alpha TE_n \Delta T} e^{\frac{TE_n}{T_{2,W}}} + F\sum_{p=1}^{P}\beta_p e^{-i2\pi\gamma B_0 f_{F,p}TE_n} e^{\frac{TE_n}{T_{2,F}}} \right) e^{-i2\pi f_B TE_n}, n=1,2,...,N$$

, wherein $S_n$ represents signal strength at an echo time $TE_n$, W represents a strength value of the water signal, F represents a strength value of the fat signal, $Y$ represents a gyromagnetic ratio, $B_0$ represents strength of the main magnetic field, a represents a temperature coefficient of a hydrogen proton in the water, P represents the number of fat peaks, a corresponding relative amplitude value and chemical shift are respectively $\beta_p$ and $f_{F,p}$, $\sum_{p=1}^{P} \beta_P = 1$,

$T_{2,W}^{*}$ represents a transverse relaxation time of the water, $T_{2,F}^{*}$ represents a transverse relaxation time of the fat, fb represents a field drift caused by the non-uniform main magnetic field, N represents the total number of collected echoes, and $\Delta T$ represents a water-fat tissue temperature image.

10. The magnetic resonance temperature imaging apparatus according to claim 9, wherein the second calculation unit is configured to fit the preset magnetic resonance signal model for the first time based on an equation

$$\left\{A_W, \Phi_W, T_{2,W}^{*}, A_F, \Phi_F, T_{2,F}^{*}, f_B, \Delta T\right\} =$$

$$\arg\min \sum_{n=1}^{N} \left( s_n - \left( We^{-i2\pi\gamma B_0 \alpha TE_n \Delta T} e^{\frac{TE_n}{T_{2,W}^{*}}} + F\sum_{p=1}^{P} \beta_p e^{-i2\pi\gamma B_0 f_{F,p} TE_n} e^{\frac{TE_n}{T_{2,F}^{*}}} \right) e^{-i2\pi f_B TE_n} \right)^2 .$$

200

101 — Memory ← Magnetic resonance temperature imaging apparatus — 100

102 — Storage controller

Peripheral interface

103 — Processor

104

FIG.1

S201 — Obtain a first strength amplitude value of a water signal, a first phase value of the water signal, a first strength amplitude value of a fat signal, a first phase value of the fat signal, a first transverse relaxation time of water, a first transverse relaxation time of fat, and a first field drift caused by a non-uniform main magnetic field, based on a preset magnetic resonance signal model, a pre-assigned initial water-fat tissue temperature image, and a water-fat separation algorithm

S202 — Obtain a second strength amplitude value of the water signal, a second phase value of the water signal, a second strength amplitude value of the fat signal, a second phase value of the fat signal, a second transverse relaxation time of the water, a second transverse relaxation time of the fat, and a second field drift caused by the non-uniform main magnetic field that minimize a difference between signal strength after the first time of fitting and signal strength before the fitting, by fitting the preset magnetic resonance signal model for the first time based on the initial water-fat tissue temperature image, the first strength amplitude value of the water signal, the first phase value of the water signal, the first strength amplitude value of the fat signal, the first phase value of the fat signal, the first transverse relaxation time of the water, the first transverse relaxation time of the fat, and the first field drift caused by the non-uniform main magnetic field

S203 — Smooth a second water-fat tissue temperature image by using a low-pass filter, to obtain a smoothed second water-fat tissue temperature image

S204 — Obtain a third strength amplitude value of the water signal, a third phase value of the water signal, a third strength amplitude value of the fat signal, a third phase value of the fat signal, a third transverse relaxation time of the water, a third transverse relaxation time of the fat, and a third field drift caused by the non-uniform main magnetic field that minimize a difference between signal strength after the second time of fitting and signal strength before the fitting, by fitting the preset magnetic resonance signal model by keeping the smoothed second water-fat tissue temperature image unchanged and based on the second water-fat tissue temperature image, the second strength amplitude value of the water signal, the second phase value of the water signal, the second strength amplitude value of the fat signal, the second phase value of the fat signal, the second transverse relaxation time of the water, the second transverse relaxation time of the fat, and the second field drift caused by the non-uniform main magnetic field

S205 — Obtain a fourth strength amplitude value of the water signal, a fourth phase value of the water signal, a fourth strength amplitude value of the fat signal, a fourth phase value of the fat signal, a fourth transverse relaxation time of the water, a fourth transverse relaxation time of the fat, a fourth field drift caused by the non-uniform main magnetic field that minimize a difference between signal strength after the third time of fitting and signal strength before the fitting, by fitting the preset magnetic resonance signal model for the third time by keeping the third phase value of the water signal, the third phase value of the fat signal, the third transverse relaxation time of the water, and the third transverse relaxation time of the fat unchanged and based on the second water-fat tissue temperature image, the third strength amplitude value of the water signal, the third phase value of the water signal, the third strength amplitude value of the fat signal, the third phase value of the fat signal, the third transverse relaxation time of the water, the third transverse relaxation time of the fat, and the third field drift caused by the non-uniform main magnetic field

FIG.2

301 302 303

| First calculation unit | — | Second calculation unit | — | Filtering unit |

| Fourth calculation unit | — | Third calculation unit |

305 304

FIG.3

<div align="center">**INTERNATIONAL SEARCH REPORT**</div>

| International application No. |
|---|
| **PCT/CN2017/119483** |

**A. CLASSIFICATION OF SUBJECT MATTER**

A61B 5/01(2006.01)i

According to International Patent Classification (IPC) or to both national classification and IPC

**B. FIELDS SEARCHED**

Minimum documentation searched (classification system followed by classification symbols)

A61B

Documentation searched other than minimum documentation to the extent that such documents are included in the fields searched

Electronic data base consulted during the international search (name of data base and, where practicable, search terms used)

CNPAT, WPI, EPODOC, CNKI, IEEE: 磁共振, 图像, 温度, 测温, 脂肪, 水, 脂, 分离, 相位, 弛豫, 时间, 场飘, 拟合, magnet +, MR , image, temperature, fat, water, transverse, relaxation, time, grease, separat+, field, float+, phase, fit+

**C. DOCUMENTS CONSIDERED TO BE RELEVANT**

| Category* | Citation of document, with indication, where appropriate, of the relevant passages | Relevant to claim No. |
|---|---|---|
| A | CN 106896334 A (TSINGHUA UNIVERSITY) 27 June 2017 (2017-06-27) description, paragraphs [0111]-[0112] | 1-10 |
| A | CN 104739409 A (SIEMENS (SHENZHEN) MAGNETIC RESONANCE CO., LTD.) 01 July 2015 (2015-07-01) description, paragraph [0117] | 1-10 |
| A | CN 105796065 A (SHENZHEN INSTITUTES OF ADVANCED TECHNOLOGY, CHINESE ACADEMY OF SCIENCES) 27 July 2016 (2016-07-27) entire document | 1-10 |
| A | CN 104382597 A (ALLTECH MEDICAL SYSTEMS LLC) 04 March 2015 (2015-03-04) entire document | 1-10 |
| A | CN 101507603 A (TSINGHUA UNIVERSITY ET AL.) 19 August 2009 (2009-08-19) entire document | 1-10 |
| A | CN 102116856 A (SHENZHEN INSTITUTES OF ADVANCED TECHNOLOGY, CHINESE ACADEMY OF SCIENCES) 06 July 2011 (2011-07-06) entire document | 1-10 |
| A | JP 2000300536 A (HITACHI MEDICAL CORP.) 31 October 2000 (2000-10-31) entire document | 1-10 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **15 August 2018** | **10 September 2018** |

| Name and mailing address of the ISA/CN | Authorized officer |
|---|---|
| **State Intellectual Property Office of the P. R. China** **No. 6, Xitucheng Road, Jimenqiao Haidian District, Beijing 100088 China** | |
| Facsimile No. **(86-10)62019451** | Telephone No. |

Form PCT/ISA/210 (second sheet) (January 2015)

## INTERNATIONAL SEARCH REPORT
### Information on patent family members

| International application No. |
| --- |
| **PCT/CN2017/119483** |

| Patent document cited in search report | | | Publication date (day/month/year) | Patent family member(s) | | | Publication date (day/month/year) |
| --- | --- | --- | --- | --- | --- | --- | --- |
| CN | 106896334 | A | 27 June 2017 | None | | | |
| CN | 104739409 | A | 01 July 2015 | CN | 104739409 | B | 13 February 2018 |
| | | | | US | 2015185304 | A1 | 02 July 2015 |
| CN | 105796065 | A | 27 July 2016 | None | | | |
| CN | 104382597 | A | 04 March 2015 | None | | | |
| CN | 101507603 | A | 19 August 2009 | CN | 101507603 | B | 14 November 2012 |
| CN | 102116856 | A | 06 July 2011 | CN | 102116856 | B | 16 January 2013 |
| JP | 2000300536 | A | 31 October 2000 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201711387804